# EUROPÄISCHE PATENTSCHRIFT

(11) **EP 0 957 838 B1**
(45) Veröffentlichungstag und Bekanntmachung des Hinweises auf die Patenterteilung: **09.08.2000**
(21) Anmeldenummer: 98965802.6
(22) Anmeldetag: 10.12.1998
(51) Int. Cl.: A61F 2/68

(54) **BEINPROTHESE MIT EINEM KÜNSTLICHEN KNIEGELENK MIT EINER REGELEINRICHTUNG**
LEG PROSTHESIS WITH AN ARTIFICIAL KNEE JOINT PROVIDED WITH AN ADJUSTMENT DEVICE
PROTHESE DE LA JAMBE COMPORTANT UNE ARTICULATION DU GENOU ARTIFICIELLE POURVUE D'UN DISPOSITIF DE REGULATION

(30) Priorität: 10.12.1997 DE 19754690
(43) Veröffentlichungstag der Anmeldung: 24.11.1999
(73) Patentinhaber: BIEDERMANN MOTECH GmbH, 78054 Villingen-Schwenningen (DE)
(72) Erfinder: BIEDERMANN, Lutz, D-78048 VS-Villingen (DE)
(74) Vertreter: Prüfer, Lutz H., Dipl.-Phys.
(86) Internationale Anmeldenummer: EP9808039
(87) Internationale Veröffentlichungsnummer: WO9929272

(56) Entgegenhaltungen:
- EP-A- 0 628 296
- WO-A-96/41599
- FR-A- 2 623 086
- US-A- 5 443 524
- US-A- 5 571 205

## Beschreibung

Die Erfindung betrifft eine Beinprothese mit einem künstlichen Kniegelenk nach dem Oberbegriff des Anspruches 1.

Beim Laufen mit einer Prothese wird der Prothesenoberschenkel durch den Beinstumpf während des Ganges nach vorn bewegt. Bei nicht angepaßter Dämpfung kann der Unterschenkel sich durch seine Massenträgheit sehr weit anwinkeln. Der Prothesenträger muß dann warten, bis sich die Prothese wieder nach vorn bewegt, bevor er deren Fuß aufsetzen kann. Damit ergibt sich ein unharmonisches Gangbild, ein ungünstiges Zeitverhalten und somit eine schlechte Trageigenschaft.

Es sind Beinprothesen mit einem künstlichen Kniegelenk bekannt, bei denen ein Dämpfungselement in Form eines Pneumatik- oder Hydraulikzylinders zur Schwungphasensteuerung und als sogenannte Rückfallbremse vorgesehen ist. Die Anpassung der Beinprothese an den Träger erfolgt dabei mittels eines stationären Ganganalysesystems. Dabei muß der Träger der Prothese einen Testlauf mit der Prothese, beispielsweise auf einem Laufband, ausführen, worauf dann ein Orthopädietechniker eine subjektive Bewertung des Gangbildes vornimmt.

Zusammen mit den subjektiven Empfindungen des Prothesenträgers wird dann eine Anpassung und Einstellung der verschiedenen Bestandteile der Prothese vorgenommen. Das Ergebnis der Einstellung ist oft ungenau, weil die Einstellung mittels subjektiver Kriterien erfolgt. Zudem werden nächträgliche Veränderungen wie die des Gewichtes, der Temperaturen bzw. der Bodenbeschaffenheit nicht berücksichtigt.

Ferner haben die bekannten Dämpfungselemente für künstliche Kniegelenke den Nachteil, daß sie nicht schnell genug auf eine abrupte Änderung der Gangdynamik reagieren können.

Aus der WO 96/41599 ist eine Beinprothese mit einem künstlichen Kniegelenk nach dem Oberbegriff des Patentanspruches 1 bekannt.

Aufgabe der Erfindung ist es, eine Beinprothese mit einem künstlichen Kniegelenk, welches eine Schwungphasensteuerung und Rückfallbremse aufweist, bereitzustellen, die einen jederzeit optimalen und an den Träger angepaßten Betrieb sowie eine schnelle Reaktion auf abrupte Änderungen der Gangdynamik gewährleistet.

Die Aufgabe wird gelöst durch eine Steuerung gemäß Patentanspruch 1. Weiterbildungen der Erfindung sind in den Unteransprüchen angegeben.

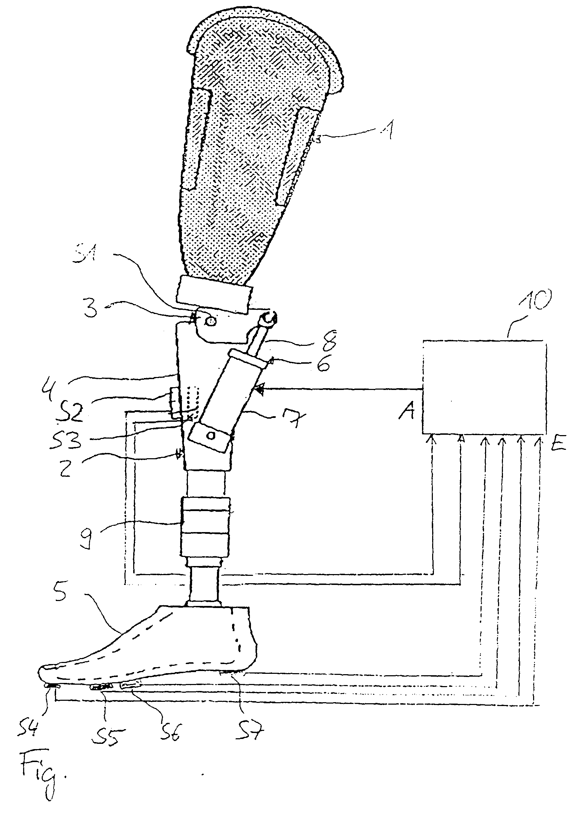

Weitere Merkmale und Zweckmäßigkeiten der Erfindung ergeben sich aus der Beschreibung eines Ausführungsbeispieles anhand der Figur.

Die Figur zeigt eine schematische Darstellung einer Beinprothese mit einem künstlichen Kniegelenk mit einer Schwungphasensteuerung und Rückfallbremse und eine dazugehörige Steuerung bzw. Regelung.

Die Prothese umfaßt in bekannter Weise ein Oberschenkelteil 1 und ein Unterschenkelteil 2 und ein die beiden verbindendes Kniegelenk 3. Das Unterschenkelteil 2 weist ein Schienbeinteil 4 mit einem Unterschenkelrohr 9 und ein mit diesem verbundenes Fußteil 5 auf. Das Fußteil 5 weist eine in der Figur nicht dargestellte Blattfeder zum Ermöglichen eines federnden Auftrittes auf. Das Oberschenkelteil 1 ist zum Verbinden mit dem Beinstumpf ausgebildet.

Das Kniegelenk 3 weist ein Dämpfungselement in Form einer hydraulischen Kolben-Zylindereinrichtung 6 auf. Der Kolben 7 der Kolben-Zylindereinrichtung 6 ist mit dem Schienbeinteil 4 verbunden und die Kolbenstange 8 der Kolben-Zylindereinrichtung 6 ist mit dem Kniegelenk 3 verbunden. Der Zylinder der Kolben-Zylindereinrichtung ist mit einer magneto-rheologische Flüssigkeit (MR Fluid) gefüllt, die die Eigenschaft aufweist, daß sich unter Einwirkung eines magnetischen Feldes ihre Viskosität im Bereich von etwa 3 bis 5 Millisekunden ändert. Die magneto-rheologische Flüssigkeit besteht aus einer Suspension von magnetisierbaren Teilchen in der Größenordnung von Mikrometern in Öl. Eine magneto-rheologische Flüssigkeit hat normalerweise eine Konsistenz ähnlich der von Motoröl. Unter Einwirkung eines magnetischen Feldes nimmt die Viskosität schlagartig zu, wobei der Grad an Änderung proportional zur Stärke des angelegten Magnetfeldes ist.

Der Kolben 8 oder der Zylinder 7 der Kolben-Zylindereinrichtung 6 weist ferner einen Elektromagneten auf, der über externe Signale ansteuerbar ist und der das Magnetfeld zum Einwirken auf die magneto-rheologische Flüssigkeit bereitstellt.

Die Beinprothese weist ferner eine Anzahl von Sensoren zur Bewegungs- und Kraftmessung auf. Im Kniegelenk 3 ist ein Kniewinkelsensor zur Erfassung des Kniewinkels vorgesehen. Am Schienbeinteil 4 sind Beschleunigungssensoren vorgesehen. Ein frontal angeordneter Beschleunigungssensor S2 dient zur Messung der Beschleunigung in Fortbewegungsrichtung, ein seitlich angeordneter Beschleunigungssensor S3 dient zur Messung der Beschleunigung senkrecht zur Fortbewegungsrichtung. Als Beschleunigungssensoren können herkömmliche Beschleunigungssensoren, wie sie beispielsweise aus der Kraftfahrzeugtechnik bekannt sind, verwendet werden. Weiterhin sind im Bereich der Fußsohle Kraftsensoren S4 bis S7 vorgesehen. Der Kraftsensor S4 ist im Zehenbereich angeordnet, die Kraftsensoren S5 und S6 sind im Fußballenbereich angeordnet und ein Kraftsensor S7 ist im Fersenbereich angeordnet. Als Sensoren können herkömmliche Kraftsensoren, beispielsweise solche auf der Basis einer Druckfeder verwendet werden. Alternativ können Kraftsensoren im Unterschenkelrohr 9 verwendet werden.

Die Signalausgänge der Sensoren S1 bis S7 sind mit einem oder mehreren Eingängen E einer Steuer- bzw. Regeleinheit 10 verbunden. Die Steuereinheit weist eine CPU und einen Datenspeicher auf. In dem Datenspeicher ist ein Programm mit einem Algorithmus zur Verarbeitung der eingehenden Signale von den Sensoren und zum Erzeugen eines oder mehrerer Ausgangssignale vorgesehen. Ein Signalausgang A der Steuereinheit 10 ist mit der Kolben-Zylindereinrichtung 6 und speziell mit dem in dem Kolben vorgesehenen Elektromagneten verbunden.

Im Betrieb arbeitet die Steuerung der Beinprothese wie folgt. Die Meßdaten der Sensoren S1 bis S7 werden an die Steuereinheit 10 geleitet. In Abhängigkeit von den Meßdaten werden durch die Steuereinheit 10 Steuersignale für die Kolben-Zylindereinrichtung erzeugt und an diese geleitet. In Abhängigkeit von den Steuersignalen wird von dem Elektromagneten ein definierten Magnetfeld erzeugt, welches eine bestimmte Viskositätsänderung der magneto-rheologischen Flüssigkeit in dem Zylinder 7 hervorruft. Durch die Änderung der Viskosität kann die Eintauchtiefe des Kolbens 8 in den Zylinder 7 und damit die Dämpfung entsprechend gesteuert werden. Die Änderung der Dämpfung erfolgt dabei innerhalb einer Zeitspanne von etwa 3 bis 5 Millisekunden. Dies ist insbesondere vorteilhaft beim Einsatz der Dämpfung als Rückfallbremse. Wenn der Träger der Beinprothese stolpert, so kann durch die sich unmittelbar aufbauende Dämpfung ein Einklappen des Unterschenkelteils frühzeitig verhindert werden.

Die Steuereinheit, die Sensoren und das Dämpfungselement sind in einem Regelkreis miteinander verbunden, d.h. es erfolgt eine Einstellung der Dämpfung während des Gehens. Dies hat gegenüber einer herkömmlichen Prothesensteuerung den Vorteil, daß die Einstellung der Prothesenfunktionen unmittelbar in Abhängigkeit von dem natürlichen Gangverhalten des Prothesenträgers erfolgt.

Abgewandelte Ausführungsformen sind denkbar. Es können weniger oder mehr als die oben beschriebenen Sensoren vorgesehen sein.

Anstelle einer Kolben-Zylindereinrichtung mit einem in dem Zylinder axial verschiebbaren Kolben kann auch eine Kolben-Zylindereinrichtung mit einem Drehkolben verwendet werden, der beispielsweise mit Schaufeln versehen ist, die in Abhängigkeit von der Viskosität der magneto-rheologischen Flüssigkeit einen bestimmten Widerstand im Zylinder erfahren. Die Kolbenstange ist dabei mit einer Drehwelle des Kniegelenks verbunden.

## Patentansprüche

1. Beinprothese mit einem künstlichen Kniegelenk mit Schwungphasensteuerung und Rückfallbremse und mit
einem an dem Kniegelenk (3) vorgesehenen Steuerelement (6), wobei das Steuerelement (6) das Kniegelenk (3) in Abhängigkeit von Steuersignalen steuert,
dadurch gekennzeichnet, daß das Steuerelement (6) als Dämpfungselement in Form einer Kolben-Zylindereinrichtung ausgebildet ist, wobei in dem Zylinder eine magneto-rheologische Flüssigkeit vorgesehen ist, die in Abhängigkeit von den Steuersignalen ihre Viskosität ändert.

2. Beinprothese nach Anspruch 1,
gekennzeichnet durch
einen an dem Bein vorgesehenen Sensor (S1 ... S7) zur Kraft- oder Kniewinkel- oder Beschleunigungsmessung,
dessen Signalausgang mit einer Steuereinheit (10) verbunden ist, die in Abhängigkeit von den gemessenen Größen die Steuersignale für das Steuerelement erzeugt.

3. Beinprothese nach einem der Ansprüche 1 und 2,
dadurch gekennzeichnet, daß
das Steuerelement (6) einen Elektromagneten aufweist, dessen Magnetfeld von den Steuersignalen abhängt.

4. Beinprothese nach einem der Ansprüche 1 bis 3,
dadurch gekennzeichnet, daß
das Dämpfungselement als Kolben-Zylindereinrichtung mit einem axial verschiebbaren Kolben oder als Drehzylindereinrichtung ausgebildet ist.

5. Beinprothese nach einem der Ansprüche 2 bis 4,
gekennzeichnet durch
einen im Kniegelenk (3) vorgesehenen Sensor (S1) zur Messung des Kniewinkels und/oder einen im Bereich des Schienbeines (S2, S3) vorgesehenen Sensor zur Messung der seitlichen bzw. frontalen Beschleunigung und/oder einen im Bereich der Fußsohle (S4, S5, S6, S7) vorgesehenen Sensor zur Messung der Kraft.

6. Beinprothese nach einem der Ansprüche 2 bis 5,
dadurch gekennzeichnet, daß
das Kniegelenk (3), die Steuereinheit (10) und das Steuerelement (6) eine Regeleinheit zur automatischen Regelung der Kniegelenksfunktionen während des Gehens in Abhängigkeit von den aktuellen Meßwerten für die Kraft und/oder die Beschleunigung und/oder den Kniewinkel bilden.

7. Beinprothese nach einem der Ansprüche 2 bis 6,
dadurch gekennzeichnet, daß
die Steuereinheit (10) am Bein angebracht ist und mit dem Steuerelement (6) fest verbunden ist.

8. Beinprothese mit einem künstlichen Kniegelenk mit Schwungphasensteuerung und Rückfallbremse nach einem der Ansprüche 2 bis 7 dadurch gekennzeichnet, daß während des Betriebes der Prothese automatisch eine Regelung der Kniegelenksfunktionen in Abhängigkeit von den von dem Sensor gemeldeten Werten erfolgt.

## Claims

1. A leg prosthesis with an artificial knee joint with oscillation-phase control and recoil brake and with
a control member (6) provided at the knee joint (3), the control member (6) controlling the knee joint (3) in dependence on the control signals,
characterised in that the control member (6) is designed as a damping member in the form of a piston-cylinder device, a magneto-rheological fluid being provided in the cylinder, the said fluid changing its viscosity in dependence on the control signals.

2. A leg prosthesis according to Claim 1,
characterised by
a sensor (S1 .... S7) provided on the leg for measuring the force, the angle of the knee, or the acceleration,
the signal output of the said sensor being connected to a control unit (10) which produces the control signals for the control member in dependence on the measured variables.

3. A leg prosthesis according to one of Claims 1 and 2,
characterised in that
the control member (6) has an electromagnet the magnetic field of which depends on the control signals.

4. A leg prosthesis according to one of Claims 1 to 3,
characterised in that
the damping member is in the form of a piston-cylinder device with an axially displaceable piston, or of a rotary cylinder device.

5. A leg prosthesis according to one of Claims 2 to 4.
characterised by
a sensor (S1) provided in the knee joint (3) for measuring the angle of the knee and/or a sensor provided in the region of the tibia (S2, S3) for measuring the lateral or frontal acceleration respectively and/or a sensor provided in the region of the sole of the foot (S4, S5, S6, S7) for measuring the force.

6. A leg prosthesis according to one of Claims 2 to 5,
characterised in that
the knee joint (3), the control unit (10) and the control member (6) form an adjusting unit for automatically adjusting the functions of the knee joint during walking in dependence on the actual measured values for the force and/or the acceleration and/or the angle of the knee.

7. A leg prosthesis according to one of Claims 2 to 6,
characterised in that
the control unit (10) is attached to the leg and is solidly connected to the control member (6).

8. A leg prosthesis with an artificial knee joint with oscillation-phase control and recoil brake according to one of Claims 2 to 7, characterised in that, during operation of the prosthesis, adjustment of the knee-joint functions occurs automatically depending on the values indicated by the sensor.

## Revendications

1. Prothèse de jambe, comportant une articulation artificielle du genou et un frein de retour, et comportant un élément de commande (6) prévu dans l'articulation du genou (3), l'élément de commande (6) commandant l'articulation du genou (6) en fonction de signaux de commande, caractérisée en ce que l'élément de commande (6), servant d'élément amortisseur, est réalisé sous forme d'un dispositif à cylindre-piston, le cylindre contenant un liquide magnéto-rhéologique qui modifie sa viscosité en fonction des signaux de commande.

2. Prothèse de jambe selon la revendication 1, caractérisée par un capteur (S1...S7) prévu sur la jambe, pour la mesure de la force, de l'angle du genou ou de l'accélération, la sortie de signal étant raccordée à une unité de commande (10) qui produit, en fonction des grandeurs mesurées, les signaux de commande pour l'élément de commande.

3. Prothèse de jambe selon l'une des revendications 1 ou 2, caractérisée en ce que l'élément de commande (6) comporte un électroaimant dont le champ magnétique dépend des signaux de commande.

4. Prothèse de jambe selon l'une des revendications 1 à 3, caractérisée en ce que l'élément amortisseur est sous forme d'un dispositif à cylindre-piston dont le piston peut se déplacer axialement ou sous forme d'un dispositif à cylindre rotatif.

5. Prothèse de jambe selon l'une des revendications 2 à 4, caractérisée par un capteur (S1) prévu dans l'articulation du genou (3), pour la mesure de l'angle du genou et/ou un capteur (S2, S3) prévu dans la zone du tibia, pour la mesure de l'accélération frontale ou latérale, et/ou un capteur (S4, S5, S6, S7) prévu dans la zone de la plante du pied, pour la mesure de la force.

6. Prothèse de jambe selon l'une des revendications 2 à 5, caractérisée en ce que l'articulation du genou (3), l'unité de commande (10) et l'élément de commande (6) constituent une unité de régulation pour la régulation automatique des fonctions d'articulation du genou pendant la marche, en fonction des valeurs de la force et/ou de l'accélération et/ou de l'angle du genou mesurées à un instant donné.

7. Prothèse de jambe selon l'une des revendications 2 à 6, caractérisée en ce que l'unité de commande (10) est placée sur la jambe et est raccordée solidement à l'élément de commande (6).

8. Prothèse de jambe comportant une articulation du genou artificielle avec régulation de la phase d'inertie et frein de retour, selon l'une des revendications 2 à 7, caractérisée en ce que, pendant le fonctionnement de la prothèse, une régulation des fonctions d'articulation du genou s'effectue automatiquement en fonction des valeurs émises par le capteur.
